# EUROPEAN PATENT SPECIFICATION

(11) **EP 0 294 189 B1**
(45) Date of publication and mention of the grant of the patent: **31.08.1994**
(21) Application number: 88305023.9
(22) Date of filing: 02.06.1988
(51) Int. Cl.: B65D 75/38, B65D 77/04, B65D 47/42, A45D 37/00

(54) **Pouch with liquid applicator and machine for making it**
Beutel mit Flüssigkeitauftragsvorrichtung und Vorrichtung für seiner Herstellung
Sachet avec un moyen d'application de liquides et machine pour le fabriquer

(30) Priority: 02.06.1987 GB 8712916; 16.07.1987 GB 8716827; 24.02.1988 GB 8804287
(43) Date of publication of application: 07.12.1988
(73) Proprietor: JAYPAK LIMITED, Alsager Stoke-on-Trent ST7 2TW (GB)
(72) Inventor: Moloney, John, Alsager Stoke-on-Trent, ST7 2TW (GB)
(74) Representative: Denmark, James

(56) References cited:
- EP-A- 0 109 737
- CH-A- 431 371
- US-A- 3 152 352
- US-A- 3 466 131
- US-A- 4 227 614
- US-A- 4 643 725

## Description

This invention is concerned with the provision of a pouch product adapted to contain liquid and including an applicator pad by means of which the liquid may be used. This product is hereinafter referred to simply as a pouch product. The invention also includes the provision of a machine by means of which a preferred pouch product may be made and filled.

The pouch product which combines a liquid holding pouch and a liquid applicator may be used as a wipe for toiletries, disinfectants, paints, cleaners and so forth and may therefore be used e.g. for applying polish to furniture, floors, motor vehicles and for other purposes.

A large number of pouch products, dispensers and applicators have been proposed in the literature, and many patent applications have been filed for these arrangements.

The Applicant is aware of a large number of prior patent specifications which disclosed these devices, and the known prior specifications to the Applicant are as follows:-

| | |
|---|---|
| European Patent Specification 0109737 | |
| U.S. Patent 4227614 | French Patent 1130737 |
| U.S. Patent 4643725 | German Patent 670101 |
| U.S. Patent 3891331 | U.S. Patent 2681168 |
| U.S. Patent 3466131 | U.S. Patent 3736933 |
| U.S. Patent 3053385 | U.S. Patent 4872556 |
| U.S. Patent 3826259 | U.S. Patent 4629080 |
| U.S. Patent 3519364 | U.S. Patent 2687130 |
| U.S. Patent 3060486 | German Patent 1198794 |
| U.S. Patent 3324855 | Swiss Patent 431371 |
| U.S. Patent 3152352 | U.K. Patent 2078897 |
| French Patent 1263715 | U.S. Patent 4430013 |

The majority of these patents are concerned with the entrapment of a quantity of liquid or paste within a unit which may be an ampoule, sachet, capsule or the like, and release of that material is effected by squeezing or breaking the unit. The material released may be applied directly to a surface, or may be injected via a hyperdermic needle or the like.

Some of the devices described in these patents also make use of an applicator pad which is a pad of an absorbent material which contains liquid, and by which the liquid may be applied to a surface.

All of these patents to a greater or lesser extent have some relevance to the present invention, but the present invention seeks to provide a pouch product which is capable of easy and continuous production, and is simple and effective to use, and in addition is inexpensive.

Because of the large amount of prior proposals, it is not straight forward to compare any one proposal directly with the present invention as being particularly relevant, but reference might be made briefly to several of the prior specifications to indicate the nature of the prior technology.

Thus, in US Patent 3466131, the dispensing package is provided in which a capsule containing a single shot or dose of a liquid product is contained inside a thermoformed bubble of plastics material which can be pressed to deform same, to rupture the capsule, and the contents of the ruptured capsule flow to an applicator pad which can in turn be applied to a surface to transfer the liquid product to the surface.

In US Patent 4629080, plastic film material is sealed in order to define two chambers one of which contains the liquid, and the other of which is empty but has an outlet defined by a nipple such as an infant nipple. This container is used in that the flexible material is squeezed to fracture a membrane and to allow the liquid to flow into the empty chamber and then to the nipple when it is desired that the material should be used.

US Patent 3060486 discloses that an absorbent pad contains in the interior thereof a capsule in which is contained the liquid material which is to be applied, and a liquid material is released by pressing the pad to rupture the capsule to allow the material to flow through the absorbent pad.

US Patent 3891331 discloses an arrangement somewhat similar to that disclosed in US patent 3466131 in that a moulded plastics outer container receives a glass ampoule in a cavity of the container. The ampoule is ruptured by pressing the container to deflect same, and liquid in the ampoule flows to an outlet of the container and thence to an absorbent pad whereby the material released from the ampoule can be applied to a surface using the container as a handle and the pad is the application surface.

In Swiss Patent 431371 an outer flexible sheet material bag contains an inner flexible sheet material sachet and the sachet contains the liquid material. Release of the liquid material is achieved by pressing the assembly so that the material flows into the outer bag, and the outer bag can then be cut to release the liquid contents.

In US Patent 3053385, a laminated construction made up of sheets of flexible plastics material defines a cavity in which a liquid is contained. The device is folded in two, and by unfolding the device rupturing of part of the sheet material creates an opening to the liquid filled cavity so that the liquid can be ejected from the cavity and can be applied to another part of the device which is an absorbent pad and the pad can then be used for application to a surface to be treated.

It can be seen therefore that the devices are many and varied, but there has been no clear indication in the prior art of a simple pouch product of the type according to the present invention, which is capable of simple production and which is effective in use, although some of the specific features of the present invention may individually be present in the prior art, such as the use of an applicator pad and flexible sheet material to define sachets and pouches.

According to the present invention there is provided a pouch product comprising a body having an interior, a sealed unit containing a liquid and disposed inside the body to occupy part only of the body interior, said unit being adapted to be ruptured by the application of finger pressure to release the liquid into the remainder of the body interior, said body being closed except for a dispensing opening and an absorbent applicator covering said opening in order to absorb said released liquid, wherein the body is a bag of flexible sheet material and the unit is a sachet of flexible sheet material.

A machine for continuously making and filling a preferred form of the pouch product includes first feeding means to feed sheet material to form the inner sachet, folding means to fold the inner sachet material to the required shape, second feeding means to feed back and front sheet material lengths to form the back and front of a bag defining said bag material, sealing means to seal the sides and the ends of the bag and to seal the inner sachet, third feeding means to supply absorbent material to form the applicator, means to secure the applicator in position and supply means to feed liquid into the inner sachet before sealing is completed.

In order that the invention may be more clearly understood reference is now directed to the accompanying drawings given by way of example in which:
Fig. 1 is a pictorial view of one form of a pouch applicator embodying the invention;
Fig. 2 is a sectional view of the pouch shown in Fig. 1;
Fig. 3 is a view of the pouch in a folded condition;
Fig. 4 shows diagrammatically a step in the manufacture of the pouch;
Fig. 5 is a front view of a finished pouch;
Fig. 6 is a rear view of the finished pouch;
Fig. 7 is a side or edge-on view of the pouch before bursting of the inner sachet;
Fig. 8 is a view similar to Fig. 7 after bursting of the inner sachet;
Fig. 9 is a view of one type of forming and filling machine;
Fig. 10 is a detail view of another type of forming and fitting machine;
Fig. 11 is a view of another type of forming and filling machine;
Figs. 12 and 13 are views of a modified form of pouch; and
Fig. 14 is a view of a modified form of pouch.

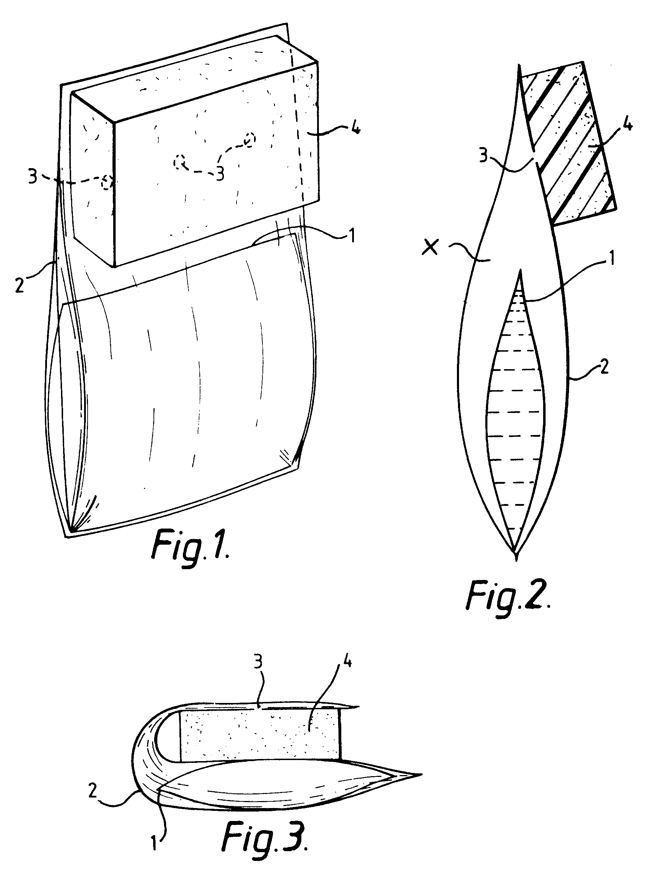

Referring first to Figs. 1 to 3, it will be seen that the pouch applicator shown includes an inner sachet 1 within the interior (X) of an outer flexible bag 2 which forms an outer sachet.

The inner sachet 1 is sealed and contains a liquid which is prevented from reaching an absorbent applicator 4 until the sachet 1 is broken. It will be noted therefore that the liquid is inside the bag 2 but the walls of the inner sachet 1 form restraint means to isolate the liquid from the bag interior (X) and the applicator (4). The bag 2 has an opening or openings 3 communicating with the rear of the absorbent applicator 4 which may for example be a sponge or an absorbent cloth.

When it is desired to use the liquid the bag 2 is squeezed thus bursting the inner sachet 1 which allows the liquid to flow into the inter of (X) of bag 2, then through the opening(s) 3 into the applicator 4. The Applicator 4 becomes impregnated with the liquid and the Applicator when in this condition may be used as desired e.g. in order to apply the liquid to a surface for cleaning or other purposes.

A pouch in accordance with this invention can be made of any suitable flexible material preferably a plastics material, the inner sachet 1 being burstable by squeezing and the bag 2 preferably being somewhat stronger than the inner sachet 1. The embodiment described above and as illustrated in Figs 1 to 3 therefore provides a pouch comprising an inner sealed flexible and burstable sachet 1 containing a liquid, an outer flexible and preferably stronger bag 2, an absorbent applicator 4 secured e.g. adhesively to the bag and opening(s) 3 in the bag 2 through which liquid in the inner sachet 1 can flow into the applicator 4 after the inner sachet 1 has been burst.

In sachet 3 the pouch is shown with the bag 2 folded round the applicator 4 after first use so that the applicator 4 is substantially enclosed by the bag material for possible further use. In this specification the term liquid is used in a broad sense to include not only liquids but also all flowable pastes that are sufficiently mobile to impregnate the applicator 4. A pouch in accordance with this invention may be made very easily on a suitable form and fill machine similar to that described in our U.K. Patent Application No 8630729, the applicator being adhered to the bag either as a separate later step or during the formation of the bag.

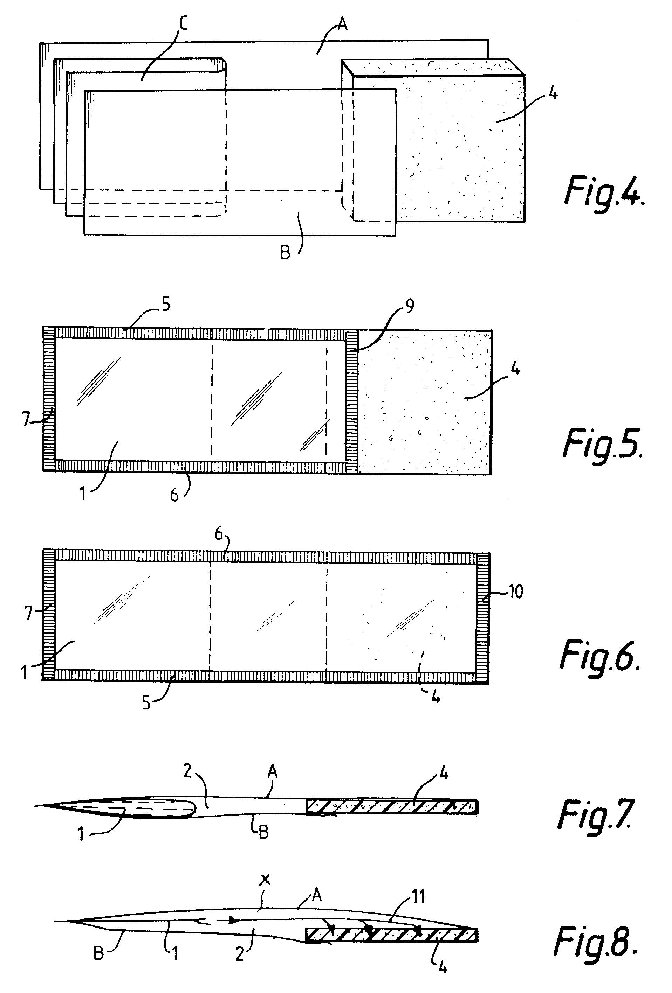

Referring next to Figs. 4 to 8 the pouch applicator is preferably made using a longer length of back material A, a short length of front material 8, folded material C and sponge or like material to form the applicator 4, sealing being effected along side edges 5, 6 and end edges 7, 9 and 10 - see Figs. 5 and 6 - to secure the applicator in position and to form the inner closed sachet 1 and the bag (2). Looking at Figs. 5 and 6, it will be seen that the applicator 4 is sealed along seal 9 and to the longer length of material along side seals 5 and 6 and along an end seal 10.

Fig. 7 shows the completed pouch applicator with the inner sachet 1 filled with liquid e.g. paint or cleaning fluid and Fig. 8 shows the pouch after bursting of the inner sachet 1 by pressure from outside the bag which permits the liquid from the inner sachet 1 to flow into the interior (X) of bag (2) and then to impregnate the applicator 4 as indicated by the arrows 11. It will be understood that the inner sachet 1 may be filled with liquid before the seal 7 or a side seal 5 or 6 is effected.

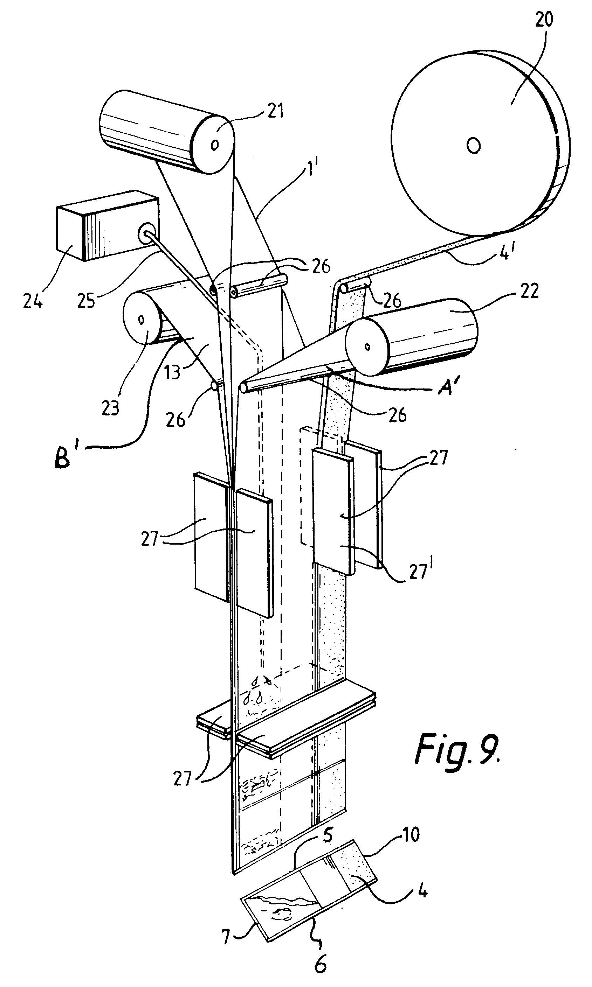

Fig. 9 shows diagrammatically one embodiment of a form and fill machine for making a pouch applicator in accordance with this invention and for filling the inner sachet 1 with liquid. In Fig. 9 applicator material 4' is fed from a roll 20 to form the applicator 4, burstable thin inner sachet material 1' is fed from a roll 21 to form the inner sachet 1 and the material 1' is folded to the shape shown in Fig. 1 to produce the inner sachet 1. Back and front longer and shorter material lengths A' and B' are fed from rolls 22 and 23 to form the back and front of the bag 2. Liquid is provided by a supply pump 24 along pipe 25. Suitable guide rolls are shown at 26, sealing is effected by reciprocable sealing elements 27 which are synchronised with intermittent forward feeding movements of the parts of the pouch.

In operation the parts of the pouch applicator are fed downwards through the machine to produce pouch applicators in the position shown in Fig. 5.

The upper set of sealing elements 27 provide the seals 7, 9 and 10 and the lower set of sealing elements provide the seals 5 and 6, liquid being fed into the inner sachet 1 through the side seal 5 after the forming of the lower edge seal 6 of each pouch and before formation of the upper edge seal 5. In the embodiment of Fig. 9 finished pouches leave the machine in the position indicated by the just formed pouch shown below the machine in Fig. 9, i.e. transverse to the direction of movement of the parts.

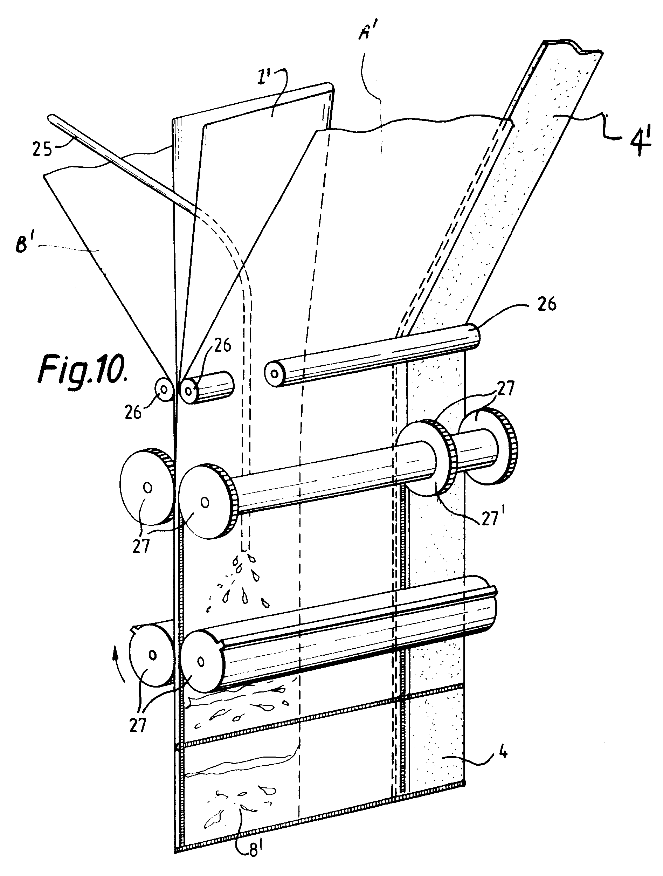

In the embodiment partially shown in Fig. 10 the feeding elements are the same as in Fig. 9 but sealing is effected by rotable sealing members 27 which can be continuously rotated as the machine is operated. It is important to note that sealing element 27'seals onoy the material A' at the front and does not attach the material B' to the applicator 4, in order to allow liquid to contact the applicator 4 when it is desired to use the liquid.

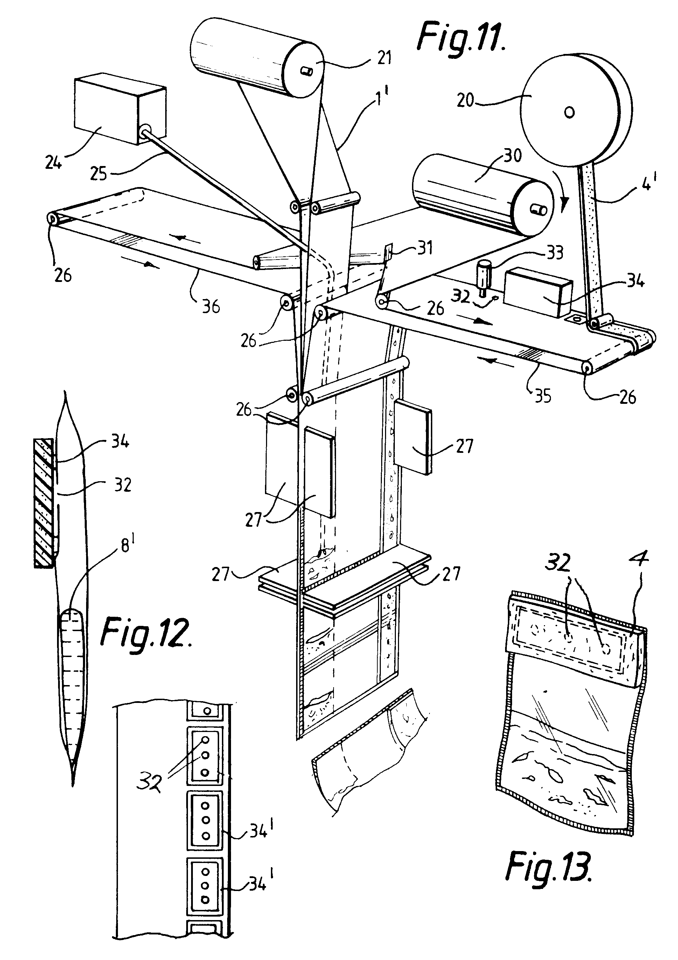

In the embodiments described in connection with Figs. 4 to 10 the applicator 4 effectively projects into the inside of the bag see e.g. Fig. 8. In Figs. 11 to 13 an embodiment of the invention is illustrated, including a machine in Fig. 11 and details of the pouch in Figs. 12 and 13, in which the applicator is secured to the outside of the pouch using e.g. hot melt glue in a box shape or like pattern, openings being punched or otherwise formed in one side prior to the glue being applied.

In Figs. 11 to 13 the same references are used for the corresponding parts as in Figs. 4 to 10. Referring first to Fig. 11 absorbent applicator material 4' is fed from roll 20, burstable inner sachet material 1' is fed from roll 21 and bag or outer sachet material is fed from roll 30, is cut by a knife 31 into two lengths 35, 36 which are fed forward over guide rolls 26. Openings 32 are punched or otherwise formed in length 35 by a hole former 33 and glue is applied by a glue applicator housed within a box like housing 34. Preferably, the flue is applied in a rectangular pattern 34' around the outside of the apertures 32 e.g. as shown in Fig. 2 so that when the application material is applied and the pouches as shown in Fig. 13 are produced, the adhesive does not restrict the flow of the liquid through the holes 32 to the applicator pad when the pouch is used.

It will be understood that the glue used must be carefully selected to be suitable for use with the liquid in the pouch so that the glue is not melted by the liquid in the inner pouch when the inner pouch is broken and the liquid is allowed to impregnate the applicator 4.

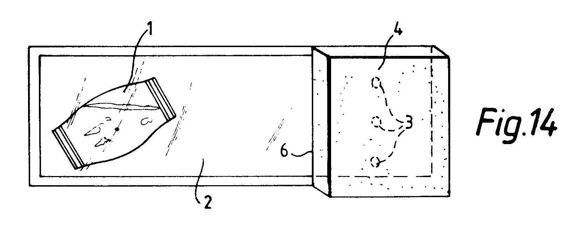

Passing on now to describe Figs. 14 to 17, Fig. 14 shows a pouch comprising a bag 2 of plastics material containing a sealed inner sachet 1 containing the liquid, the sachet 1 being freely positioned within the interior bag 2. Sponge or like absorbent material is connected to the bag, as shown, to provide the applicator 4 and openings 3 are provided as before. The sealed inner sachet 1 is therefore loosely or freely held within the bag 2 and the sealed sachet 1 forms restraint means so that the liquid is kept away from the applicator 4 until the sachet 1 is burst by pressure from outside the bag whereupon liquid can flow into the applicator 4 which is sealed along the outer edges but not along edge 6.

In Figs. 15 and 16, the sheet material which defines the bag interior (X) also defines the sachet (1) which contains the liquid.

In Fig. 15 the liquid is held within a sachet 2b, and is isolated from the bag material (X) and the applicator 4 by restraint means in the form of a clip 8. Removal of the clip 8 allows liquid to flow from sachet 2b into the interior (X) of the bag material (X) to reach the applicator (4).

In Fig. 16 the restraint means in the form of a seal 8' isolates the inner sachet 1a from the bag interior (X) and thus from the applicator until the seal 8' is broken by outside pressure. The embodiment of Gis. 17 is the same as the embodiment of Fig. 14 except that a flexible bottle-shaped sealed inner sachet is used to restrain the liquid instead of a barrel shaped sealed inner sachet 1 as in Fig. 14. In the Fig. 17 embodiment outside pressure is used to eject a stopper from the inner sachet 1 instead of bursting the sachet.

## Claims

1. A pouch product comprising a body (2) having an interior (X), a sealed unit (1) containing a liquid and disposed inside the body (2) to occupy part only of the body interior (X), said unit (1) being adapted to be ruptured by the application of finger pressure to release the liquid into the remainder of the body interior (X), said body (2) being closed except for a dispensing opening (3) and an absorbent applicator (4) covering said opening in order to absorb said released liquid, wherein the body is a bag (2) of flexible sheet material and the unit (1) is a sachet (1) of flexible sheet material.

2. A pouch product according to claim 1, characterised in that the bag (2) is rectangular and is made up of sections of flexible sheet material sealed together at the side edges (5, 6).

3. A pouch product according to claim 2, characterised in that the sheet sections of the bag (2) are sealed together at one end (7) and are open at the other end (9) and the applicator (4) projects into the bag (2) through said open end (9).

4. A pouch product according to claim 2, characterised in that the bag (2) is closed at its sides (5,6) and ends and the opening is in a wall thereof, and the applicator (4) is secured to said wall externally of the bag (2) so as to cover the opening (3).

5. A pouch product according to claim 3 characterised in that at said open end (9) one sheet section of the bag (2) projects beyond the other and the applicator (4) is secured to the projecting portion of said one sheet section of the bag (2).

6. A pouch product according to any of the claims 2 to 5, characterised in that the sachet (1) is defined by a single additional section of flexible sheet material folded in two and sealed between the bag side edges (5,6).

7. A pouch product according to any preceding claim 2 to 6, characterised in that the sachet (1) is located at one end of the bag (2) and the opening (3) and applicator (4) are located at the other end.

8. A machine for continuously making pouch products according to claim 1, characterised in that the machine comprises first feeding means (21) to feed sheet material (11) to form the inner sachet (1), folding means (26) to fold the inner sachet material (11) to the required shape, second feeding means (22, 23) to feed back and front sheet material lengths (A', B') to form the back and front of a bag defining said bag material (X), sealing means (27) to seal the sides and the ends of the bag (25) and to seal the inner sachet (1), third feeding means (20) to supply absorbent material (41) to form the applicator (4), means (27) to secure the applicator (4) in position and supply means (24, 25) to feed liquid into the inner sachet (1) before sealing is completed.

9. A machine according to claim 8, characterised in that the sheet material to form the bag (2) is fed from a roll (30) and is divided (e.g. by knife 31) into two lengths (35,36) which are fed forwards over guide rolls (26), openings (32) being formed in length (35) by a hole former (33) and glue being applied by a glue applicator device (34) to secure the pad (4) in position.

## Patentansprüche

1. Beutelförmiges Erzeugnis, mit einem Tragkörper (2), welcher einen Innenraum (X) aufweist mit einem, versiegelten Behältnis (1), welches eine Flüssigkeit enthalt und innerhalb des Tragkörpers (2) angebracht ist und dabei nur einen Teil des Innenraums (X) des Tragkörpers (2) einnimmt, mit einer solchen Ausbildung des Behältnisses (1), daß es bei Anlegen von Fingerdruck aufreißt und die Flüssigkeit für den restlichen Innenraum (X) des Tragkörpers (2) freigibt, wobei der Tragkörper (2) bis auf eine Spenderöffnung (3) geschlossen ist, und mit einem absorbierenden Applikationsorgan (4), welches die Spenderöffnung (3) bedeckt, um die freigesetzte Flüssigkeit zu absorbieren, **dadurch gekennzeichnet**, daß der Tragkörper eine Tasche (2) aus elastischem Folienmaterial und das Behältnis (1) ein Kissen (1) aus elastischem Folienmaterial ist.

2. Beutelförmiges Erzeugnis nach Anspruch 1, **dadurch gekennzeichnet**, daß die Tasche (2) rechteckförmig und aus Abschnitten von elastischem Folienmaterial hergestellt ist, welche an den Seitenkanten (5,6) zusammengeschweißt sind.

3. Beutelförmiges Erzeugnis nach Anspruch 2, **dadurch gekennzeichnet**, daß die Folienabschnitte der Tasche (2) an einem Ende (7) zusammengeschweißt und an dem anderen Ende (9) offen sind, und daß das Applikationsorgan (4) in die Tasche (2) durch das offene Ende (9) hineinragt.

4. Beutelförmiges Erzeugnis nach Anspruch 2, **dadurch gekennzeichnet**, daß die Tasche (2) an ihren Seiten (5, 6) und Enden geschlossen ist und sich die Spenderöffnung (3) in einer Wand der Tasche (2) befindet, und daß das Applikationsorgan (4) an dieser Wand außerhalb der Tasche (2) derart befestigt ist, daL` es die Öffnung (3) bedeckt.

5. Beutelförmiges Erzeugnis nach Anspruch 3, **dadurch gekennzeichnet**, daß an dem offenen Ende (9) ein Folienabschnitt der Tasche (2) über den anderen Folienabschnitt übersteht, und daß das Applikationsorgan (4) an dem überstehenden Teil des einen Folienabschnitts der Tasche (2) befestigt ist.

6. Beutelförmiges Erzeugnis nach einem der Ansprüche 2 bis 5, **dadurch gekennzeichnet**, daß das Kissen (1) durch einen einzigen zusätzlichen Abschnitt des elastischen Folienmaterials gebildet wird, welcher in zwei Teile gefaltet und zwischen den Seitenkanten (5, 6) der Tasche (2) versiegelt ist.

7. Beutelförmiges Erzeugnis nach einem der Ansprüche 2 bis 6, **dadurch gekennzeichnet**, daß das Kissen (1) an einem Ende der Tasche (2) angeordnet ist, und daß die Spenderöffnung (3) und das Applikationsorgan (4) an dem anderen Ende der Tasche (2) angeordnet sind.

8. Maschine zur kontinuierlichen Herstellung von beutelförmigen Erzeugnissen nach Anspruch 1, **dadurch gekennzeichnet**, daß die Maschine folgende Merkmale aufweist:
- Eine erste Zuführstation (21), welche Folienmaterial (11) zur Herstellung des innenliegenden Kissens (1) zuführt;
- Eine Faltstation (26), welche das Kissenmaterial (11) in die erforderliche Form faltet;
- eine zweite Zuführstation (22, 23), welche rückseitige und vorderseitige Folienmaterialbahnen (A', B') zur Herstellung der Rückseite und Vorderseite einer Tasche zuführt, welche den Tragkörper-Innenraum (X) umgrenzt,
- eine Versiegelungsstation (27), welche die Seiten und Enden der Tasche (2) sowie das innenliegende Kissen (1) versiegelt,
- eine dritte Zuführstation (20), welche absorbierendes Material (41) zur Herstellung des Applikationsorgans (4) zuführt,
- eine Einrichtung (34) zum Befestigen des Applikationsorgans (4) in der gewünschten Lage, und
- eine Versorgungseinrichtung (24,25) zum Zuführen von Flüssigkeit in das innenliegende Kissen (1), bevor dessen Versiegelung beendet ist.

9. Maschine nach Anspruch 8, **dadurch gekennzeichnet**, daß das Folienmaterial zur Herstellung der Tasche (2) von einer Rolle (30) zugeführt und in zwei Materialbahnen (35, 36) (z.B. mittels eines Messers 31) geteilt wird, die über Führungsrollen (26) in Vorwärtsrichtung abgezogen werden, daß Öffnungen (32) in der Materialbahn (35) mittels eines Lochers (33) gebildet werden, und daß Klebstoff mittels einer Klebstoff-Auftragvorrichtung (34) aufgetragen wird, um das Applikationsorgan (4) in der gewünschten Lage zu befestigen.

## Revendications

1. Produit du type trousse comportant un corps (2) présentant un intérieur (X), une unité scellée (1) contenant un liquide et disposé dans le corps (2) de manière à occuper une partie seulement de l'intérieur (X) du corps, ladite unité (1) étant adaptée pour se rompre en appliquant une pression des doigts afin de libérer le liquide dans le reste de l'intérieur (X) du corps, ledit corps (2) étant fermé à l'exception d'une ouverture d'évacuation (3.), et un applicateur absorbant (4) recouvrant ladite ouverture afin d'absorber ledit liquide libéré, dans lequel le corps est un sac (2) en matière souple en feuille et l'unité (1) est un sachet (1) en matière souple en feuille.

2. Produit du type trousse suivant la revendication 1, caractérisé en ce que le sac (2) est rectangulaire et fait de portions de matière souple en feuille scellées ensemble par leurs bords latéraux (5, 6).

3. Produit du type trousse suivant la revendication 2, caractérisé en ce que les portions de feuille du sac (2) sont scellées ensemble à un bout (7) et ouvertes à l'autre bout (9), et en ce que l'applicateur (4) fait saillie dans le sac (2) par ledit bout ouvert (9).

4. Produit du type trousse suivant le revendication 2, caractérisé en ce que le sac (2) est fermé sur ses bords (5, 6, et à ses extrémités, en ce que l'ouverture est dans une de ses parois, et en ce que l'applicateur (4) est fixé à ladite paroi extérieurement au sac (2) de façon à recouvrir l'ouverture (3).

5. Produit du type trousse suivant la revendication 3, caractérisé en ce que, audit bout ouvert (9), une première portion en feuille du sac (2) est en saillie de l'autre côté de la seconde portion et en ce que l'applicateur (4) est fixé à la partie en saillie de ladite première portion en feuille du sac (2).

6. Produit du type trousse suivant l'une des revendications 2 à 5, caractérisé en ce que le sachet (1) est défini par une seule portion supplémentaire de matière souple en feuille pliée en deux et scellée entre les bords latéraux (5, 6) du sac.

7. Produit du type trousse suivant l'une des revendications précédentes 2 à 6, caractérisé en ce que le sachet (1) est situé à un bout du sac (2) et en ce que l'ouverture (3) et l'applicateur (4) sont situés à l'autre bout.

8. Machine pour fabriquer d'une manière continue des produits du type trousse suivant la revendication 1, caractérisée en ce que la machine comprend un premier moyen d'alimentation (21) pour fournir la matière en feuille (11) afin de former le sachet interne (1), un moyen de pliage (26) pour plier la matière du sachet interne (11) à la forme requise, un deuxième moyen d'alimentation (22, 23) pour fournir des longueurs de matière en feuille, arrière et avant (A', B'), pour former l'arrière et l'avant d'un sac définissant ladite matière (X) du sac, un moyen de scellement (27) pour sceller les côtés et les bouts du sac (25) et pour sceller le sachet interne (1), un troisième moyen d'alimentation (20) pour fournir la matière absorbante (41) afin de former l'applicateur (4), un moyen (27) pour fixer l'applicateur (4) en position et un moyen d'alimentation (24, 25) pour envoyer du liquide dans le sachet interne (1) avant que le scellement soit terminé.

9. Machine suivant la revendication 8, caractérisée en ce que la matière en feuille destinée à former le sac (2) soit alimentée par un rouleau (30) et soit divisée (par exemple par un couteau 31) en deux longueurs (35, 36) qui sont avancées sur des rouleaux de guidage (26), des ouvertures (32) étant formées dans la longueur (35) par un poinçon (33) et de la colle étant appliquée par un dispositif applicateur de colle (34) pour fixer le tampon (4) en position.
